# EUROPEAN PATENT APPLICATION

(11) **EP 4 692 113 A1**
(43) Date of publication of application: **11.02.2026**
(21) Application number: 25758218.9
(22) Date of filing: 09.01.2025
(51) Int. Cl.: C07K 14/435, A01K 67/60, A01N 63/14, A01N 63/50, A01P 3/00, A23K 20/147, A23K 20/195

(54) **METHOD AND DEVICE FOR INDUCING ANTIMICROBIAL PEPTIDE OF FLY**

(30) Priority: 20.02.2024 JP 2024023711
(71) Applicant: E'S Inc., Tokyo 105-0004 (JP)
(72) Inventor: KITAZUMI Kazushige, Tokyo 105-0023 (JP); YASUMATSU Junji, Kawasaki-shi Kanagawa 211-0064 (JP); TANAKA Kimihiro, Minamisatsuma-shi Kagoshima 897-0003 (JP); HOSHINO Hitoshi, Tokyo 140-0015 (JP)
(74) Representative: Patent 42
(86) International application number: PCT/JP2025/000586
(87) International publication number: WO 2025/177721

(57) **Abstract**

[Problem] A method for inducing antibacterial peptides in insect's body.

[Solution method] In a method for inducing antibacterial peptides in insects by damaging the insects, the method is characterized in that the damaging of the insects is performed by irradiation of a laser beam, in such a manner that the laser beam penetrates the insect's epidermis to make a hole in the insects without causing irreparable damage to the insect, so that antibacterial peptides is induced in the insects. An apparatus for inducing antibacterial peptides in a body of insect by the method according to claim 1, comprising a CO₂ laser irradiation unit, a position information acquisition means for obtaining a position information of the insect, and a means for transmitting the insect position information obtained by the position information acquisition means to the insect position information. Use of a CO₂ laser in a method for inducing antibacterial peptides in the insect's body by making holes that penetrate the insect's epidermis.

## Description

### [Technical field]

The present invention relates to method and apparatus for inducing (producing) antimicrobial peptides in insects such as house flies.

### [Background technology]

A term "antibacterial peptide" is a general term for peptides formed by chained amino acids and have antibacterial effects against bacteria, viruses, fungi, etc., and are expected to lead to the development of new antibacterial drugs and epidemic prevention methods. In fact, feed additives containing antibiotics have led to the emergence of super bacteria that are resistant to antibiotics used in animals or humans, and restrictions on their use have been tightened. Therefore, there is a demand for the development of antimicrobial peptides as feed additives that provide safe and environmentally friendly alternatives to antibiotics.

[Patent Document 1] (Japanese Patent No. 3,661,549) describes a peptide segment of 26-kDa protease that exhibits antibacterial activity derived from *Boettcherisca peregrina* and an antibacterial agent containing the same. This polypeptide is a protease that appears only in the intestine during the mid-pupal stage of the fly larva and is a linear polypeptide consisting of 239 amino acids that exhibit selective antibacterial activity against Gram-positive bacteria.

To induce such antimicrobial peptides, it has been used for a long time to injure living organisms such as insects. For example, [Patent Document 2] (Japanese Patent No. 2,671,912) discloses a technique to induce an antibacterial protein in the body fluid of a Formosan rhinoceros beetle larva by wounding its body surface. [Non-patent Document 1] (Kawasaki et al. Drug Discoveries & Therapeutics 2017, page 1-5, Advance Publication) describes such a fact that, when house fly larvae were injured with a needle, body fluids collected therefrom exhibit clear antibacterial activity against both of Gram-positive bacteria such as Staphylococcus aureus and Pseudomonas aeruginosa and Gram-negative bacteria, and It has been confirmed that the antibacterial activity is predominantly retained in body fluids even after heat treatment. They showed that injured or wounded larvae can be a medical resource for antibacterial agents.

The wounding procedure, however, is generally inefficient and labor-intensive, because it requires the needle to be pierced individually, i.e., each insect. In fact, the process for injuring insects is inefficient and time-consuming work. Still more, it is a labor-intensive task to properly wound all of them with a needle because they weigh only 25 mg per fly even house fly larvae reach their maximum size just before they pupate. Further more, the induction effect differs depending on how the wound is made. In fact, the induction effect differs depending on which part of the house fly larva is inserted with the needle. The mechanism by which antimicrobial peptides are induced has not yet been precisely elucidated, although there are reports that stimulating the fat body is sufficient.

[Patent Document 3] [Patent Document 3] (Japanese Patent Application Publication No. 2009-268448) discloses a device for producing antibacterial peptides by the steps comprising isolating centipede fly from food while maintaining moisture, anesthetizing them under cool and injuring them with needle to produce antimicrobial peptides. In this patent, a large number of third-instar larvae on a tray are image-processed to determine the positional information of each third-instar larva, and a needle that is driven up and down is used to inflict stings on each third-instar larva from directly above. In this case, the sting is carried out such a manner that a needle penetrating almost through the larva. It is said that the wound closes within a few minutes due to its self-healing ability, so that no body fluids leak.

However, because the protein that makes up the larvae's epidermis is hard, a tip of the needle will curl after about 10 larvae are injured with a needle. After then, if the sting is carried out forcefully, there is a danger that the larva will die. For this reason, the needles had to be replaced frequently, so that the labor and expense hindered mass production. Therefore, [Patent Document 4] (Chinese Patent No. CN1144597) uses a different way, instead of wounding fly larvae with needles, to produce antibacterial peptides by genetical modified in which fly larvae are genetically modified and the genetically modified fly larvae are used to induce (produce) antibacterial peptides.

The present inventors have tried to find a method that is less costly than the method of injuring larvae with a needle and have tried various methods such as "a method for dropping hydrochloric acid or sulfuric acid onto the larvae", " a method for stimulating the larvae with the hyphae and spores of mushrooms that parasitize the larvae" and "a method of applying stress to the larvae due to rapid temperature changes". However, no method has been found to effectively induce antimicrobial peptides in house fly larvae.

The present inventor had thought that it would be possible to induce antibacterial peptides effectively in house fly larvae by wounding the larvae with laser irradiation. However, simply irradiating the house fly larvae with a laser resulted in complete annihilation of the house fly larvae. Therefore, the inventor thought that that this was due to the laser output being too high and repeated experiments with lower laser irradiation output, resulting in the survival rate of the laser irradiation succeeded to increase. However, the house fly larvae did not induce antibacterial peptides. Namely, the survival rate can be increased but antibacterial peptides cannot be induced when the house fly larvae were irradiated with lower laser irradiation output.

### [Prior art documents]

### [Patent documents]

[Patent Document 1] Japanese Patent No. 3661549
[Patent Document 2] Japanese Patent No. 2671912
[Patent Document 3] Japanese Patent Application Publication No. 2009-268448
[Patent Document 4] Chinese Patent No. CN1144597

### [Non-patent literature]

[Non-patent Document 1] Kawasaki et al. Drug Discoveries & Therapeutics 2017, page 1-5, Advance Publication

### [Summary of the invention]

### [Problem to be solved by the invention]

An object of the present invention is to provide a method for inducing (producing) antimicrobial peptides efficiently in housefly larvae, with keeping high survival rate of housefly larvae when the housefly larvae are irradiated with a laser.

Another object of the present invention is to provide an antimicrobial peptide production method and apparatus that can increase the production efficiency of antimicrobial peptides.

Yet another object of the present invention resides in use of a laser in the efficient induction (production) of antibacterial peptides in house fly larvae.

### [Means to solve the problem]

The first subject of the present invention resides in a method for inducing antibacterial peptides in insects by damaging the insects, characterized in that the damaging of the insects is performed by irradiation of a laser beam, in such a manner that the laser beam penetrates the insect's epidermis to make a hole in the insects without causing irreparable damage to the insect, so that antibacterial peptides is induced in the insects.

In a preferred embodiment of the present invention, the insect is a house fly larva, the laser is a CO₂ laser, and the diameter of the hole penetrating the epidermis of the house fly larva is 1 to 300 µm.

In a preferred embodiment of the present invention, the laser is irradiated to the insect larva under such a condition that movement of the insect larva is sealed by lowering a temperature just before freezing.

In a preferred embodiment of the present invention, after laser irradiation, the house fly larva irradiated with the laser is transferred to an environment at a bulb temperature of 20°C to 35°C for 3 hours to 48 hours to induce antibacterial peptides in the larva.

In a preferred embodiment of the invention, the house fly larvae are placed on a wet surface containing a liquid with a liquid having a near osmotic pressure maintained at 20°C. to 35°C so that the house fly larvae is contacted with the liquid to inhibit pupation.

The second subject of the present invention is an apparatus used to induce antibacterial peptides in a body of insect by the method according to the present invention, comprising a CO₂ laser irradiation unit, a position information acquisition means for obtaining a position information of the insect, and a means for transmitting the insect position information obtained by the position information acquisition means to the insect position information.

In a preferred embodiment of the present invention, the apparatus comprising further a means to prevent movement of the insect larvae by cooling the insect larvae to a temperature just below freezing.

A third object of the invention is a use of a CO₂ laser in a method to induce antibacterial peptides in the insect's body by making holes that penetrate the insect's epidermis.

### [Brief Description of Drawings]

[Fig. 1] is a schematic illustration for explaining the method of the present invention.
[Fig. 2] is a schematic plane view illustrating how a group of housefly larvae are irradiated with a laser beam according to the present invention.
[Fig. 3] is a schematic illustration showing an image processing device which can be used in the method for inducing the antibacterial peptide according to the present invention.
[Fig. 4] is a conceptual drawing showing the positional information of a group of house fly larvae obtained by binarizing camera-captured images of house fly larva groups
[Fig. 5] is a flow diagram of image processing used in an embodiment of the image processing system.
[Fig. 6] is a screen display of the abdominal position of respective house flies obtained from precise position information.
[Fig. 7] is a conceptual drawing of the image processing for identifying the abdomen of each house fly.

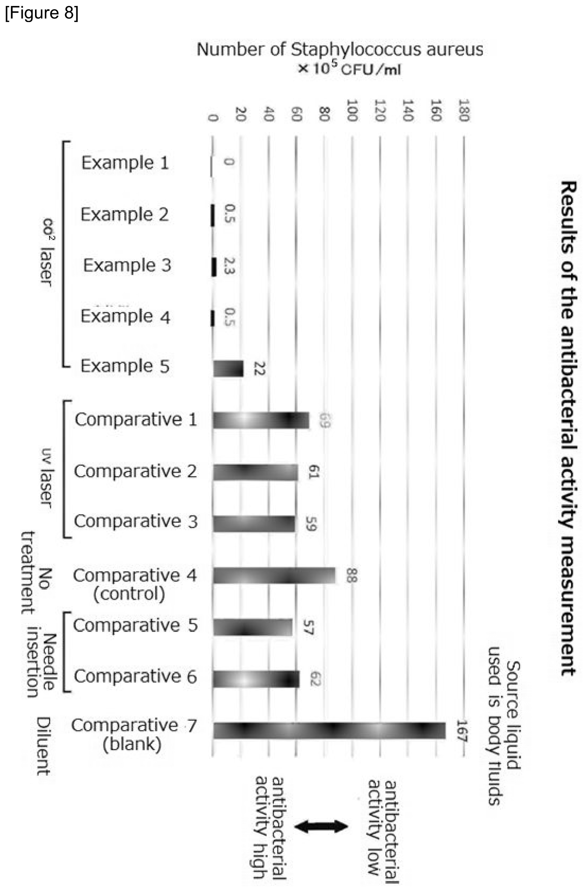

[Fig. 8] is a graph showing the results of antibacterial activity measurement in Examples and Comparative Examples, in which the vertical axis of the graph indicates the colony forming units (CFU) for staphylococcus aureus.
[Fig. 9] is a photograph showing that a hole of about 250 µm was formed in an epidermis of a house fly larva irradiated with a laser beam in Example 1.
[Fig. 10] is a photograph showing that no holes are made in the epidermis of the house fly larva when a UV laser beam is used in Comparative Example 5.

### [Description of the Preferred embodiments]

Term "Insects" generally refers to arthropod insects, but in this specification, it is meant to include a variety of small animals, including earthworms, spiders, centipedes, snails, etc., without adhering to strict academic classification. The present invention is preferably applicable to flies, black soldier flies, mealworms, crickets, silkworms, etc., and is particularly preferably applicable to flies, black soldier flies, mealworms, etc.

In this specification, the present invention will be explained using the house fly (Musca domestica) as an example, but the present invention is not limited to the house fly, and as long as the method of the present invention is applicable, it can be applied to any insects. Although insect larvae will be described, the method of the present invention can be applied not only to insect larvae but also to insect pupae and adults.

It is thought that house flies suppress the growth of fungi through competition for nutrients with Gram-negative bacilli that live symbiotically on their surfaces, and that they induce antibacterial peptides when their bodies are damaged. Therefore, damaging the body can stimulate the production of antimicrobial peptides.

In the present invention, irradiation of a laser beam is performed "without causing irreparable damage to the insect" or under such a condition that the insect does not die before the antibacterial peptide is induced. In the method of the present invention, an antimicrobial peptide induction time of 3 to 48 hours is required after laser irradiation. It is desirable that the insects do not die during this antimicrobial peptide induction time. After irradiation, the insects are subjected generally to preservation measures such as drying, freezing or refrigerating, and then they are provided as feed. Usually, the insects are not pupated because the antibacterial peptides may disappear if they are pupated.

### Preparation of insects

Housefly larvae can be supplied by a method described in the applicant's patent (Patent Document 5, Patent No. 5,579,122).
[Patent Document 5] Japanese Patent No. 5,579,122

Numerous patent documents describe how to grow and rear insects such as earthworms, mealworms, crickets, and silkworms to which the method of the present invention is applicable.

In the method of the present invention, larvae is obtained from eggs laid by adult house flies, is glowed to the 3rd instar larvae before pupal metamorphosis. In Patent Document 5, antibacterial peptides are produced by abrading the skin of larvae. According to the present invention, antibacterial peptides are produced by making holes in the skin of larvae by laser irradiation.

### Laser irradiation

A condition of "the laser beam penetrates the insect's epidermis to make a hole in the insects" according to the present invention means that a laser beam actually creates a hole through the insect's epidermis, rather than simply applying stress to the insect by the irradiation of a laser beam. The type of laser, the intensity of the laser beam and the irradiation time for making a hole through the insect's epidermis can be determined easily through experiments and depends on the type of insect and the type of laser irradiation device used.

In the case of adult house flies, the size of the hole penetrating the epidermis of the insect is generally in the range of 1 to 300 µm, preferably in the range of 10 to 250 µm. If it is less than 1 µm, the effect of the present invention cannot be expected, and if it exceeds 300 µm, the damage to adult house flies will be great.

Any type of "laser" can be used as long as it can make a hole that penetrates the insect's epidermis, but CO₂ lasers are generally used. Various laser irradiation devices are commercially available. In the present invention, commercially available devices can be preferably laser markers used to engrave (print) marks on various works. In particular, in the present invention, a laser marker that can automatically control the laser beam along three axes according to the three-dimensional shape of the insect as a workpiece can be advantageously used. Such laser markers are commercially available from Keyence Corporation, for example.

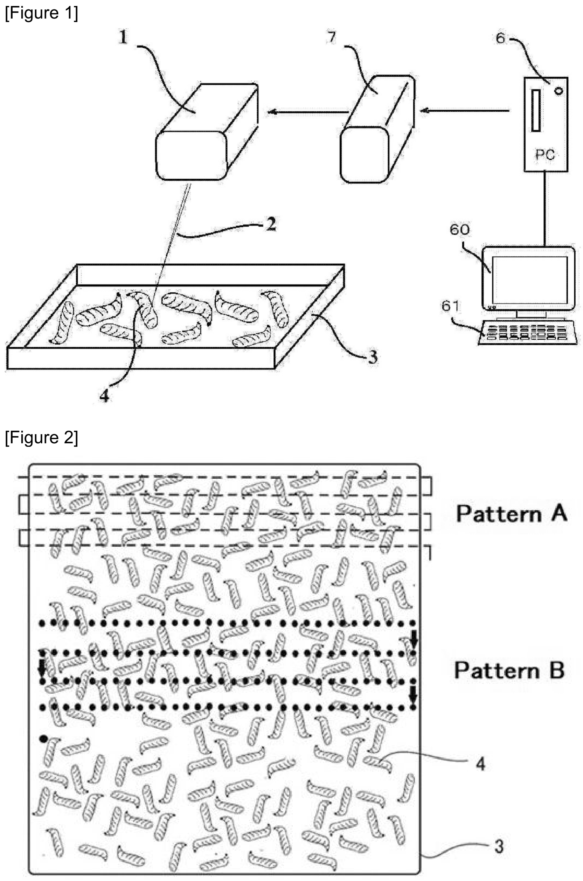

[FIG. 1] is a conceptual drawing for explaining the method of the present invention when a commercially available laser marker is used. Generally, the method of the present invention can be carried out by forming holes (that is, printing desired symbols) into a large number of insects (4) placed in a container such as a tray (3) successively by scanning a laser beam (2) from a head (1) horizontally, vertically and horizontally within an X-Y plane. In actual use, it is sufficient to input various necessary parameters (laser output, scan speed, pulse frequency, etc.) into the computer (6) through the monitor (60) and keyboard (61). It is preferable that the setting of various parameters to follow recommendation of the manufacturer of the laser marker device.

A group of housefly larvae (4) placed in a container (3) move around actively, so the position of each insect changes over time, making it difficult to know the exact location of each insect. However, in the present invention, it is only necessary for the laser beam to hit the house fly larvae (4). In fact, when the laser beam from the device shown in Figure 1 is irradiated at a predetermined interval and scanned, at least some parts of the housefly larvae (4) are hit by the laser beam. In this case, some larvae (4) will be hit by the laser beam multiple times, while some larvae (4) will not be hit by the laser beam. It is possible to find and select such optimal scanning and irradiation conditions that the probability of hitting the house fly larva (4) will be increased by experiments.

[FIG. 2] illustrates two typical marking patterns (pattern A, pattern B) for carrying out the method of the present invention. Pattern A in FIG. 2 is a conceptual diagram of a method in which a laser beam is irradiated (scanned) in a line segment pattern of a constant length, and pattern B is a conceptual diagram in which a laser beam is irradiated in a dot pattern. A hole that penetrates the insect's epidermis can be created by using a laser beam of a suitable marking pattern. Commercially available laser markers are capable of marking (printing) a desired mark (symbol) on a workpiece by irradiating a laser beam continuously or in a pulsed manner, so the method of the present invention can be easily implemented using a commercially available laser marker.

As mentioned above, in the case of house fly larvae, the third instar larvae move around actively, so it is not easy or possible to mark the exact location of each larvae. In other words, the position of the insect (work) changes over time. Therefore, it is preferable to stop the movement of the larva during laser irradiation.

Although the method of stopping the movement of the larvae is arbitrary, generally the movement of the house fly larva can be stopped by cooling it until just before it freezes. The movement of larvae can also be stopped by using chemicals, such as anesthetics such as CO₂ gas, or by physical restraint in addition to taking advantage of this temperature change.

When the movement of house fly larvae is stopped by cooling them until just before they freeze, the larva will die if it freezes, but the movement of the larva will slow down considerably at temperatures below 10°C. Therefore, it is possible to damage the larvae by irradiating the laser beam as evenly as possible by cooling to a temperature below 10°C to prevent the movement of house fly larvae. In practice, the movement of the larvae can be stopped, or almost stopped, by arranging a group of third-instar larvae on a flat surface in a tray that has been cooled to about 3°C in a refrigerator. It is possible to make holes as evenly as possible in a plurality of house fly larvae by scanning and irradiating a group of house fly larvae (4) that have stopped moving with a laser beam. It is preferable to perform test printing first and adjust the hole positions and intervals.

The intensity and irradiation time of the laser beam depend on the type of laser irradiation device used and the type of insect, and can be determined through experiments. For example, when using a CO₂ laser marker with a wavelength of 10.6 µm and an output of 30 W, it is possible to make a hole that penetrates the epidermis of a house fly larva by applying heat equivalent to 0.0096 J to one house fly larva. The intensity and irradiation time of the laser beam can be determined experimentally depending on the laser irradiation device used. The CO₂ laser uses heat to create holes in the epidermis of house fly larvae. Although the effect of this hole-opening on the body of the house fly larva is currently unknown, the laser irradiation method of the present invention does not kill the house fly larva and effectively induces antibacterial peptides.

If the laser scanning irradiation method is used without precisely detecting the location of house fly larvae, some house fly larvae may die depending on where the laser beam hits. For example, house fly larvae will die if a laser beam hits a vital part of the body, such as the heart. The actual survival rate will be as high as 80%. In this case, dead house fly larvae may be removed if necessary, but they may also be provided as feed product without being removed.

### Image processing

If a commercially available laser marker is used as is, it is not possible to precisely obtain or measure the position information of the insect (workpiece), so some insects (workpieces) may not be hit by the laser beam, or some insects (workpieces) may be hit by multiple laser beams. Further, the laser beam may be irradiated to a place where there are no insects (workpieces). Therefore, the efficiency of the method of the present invention can be improved by processing the image of the insect (workpiece).

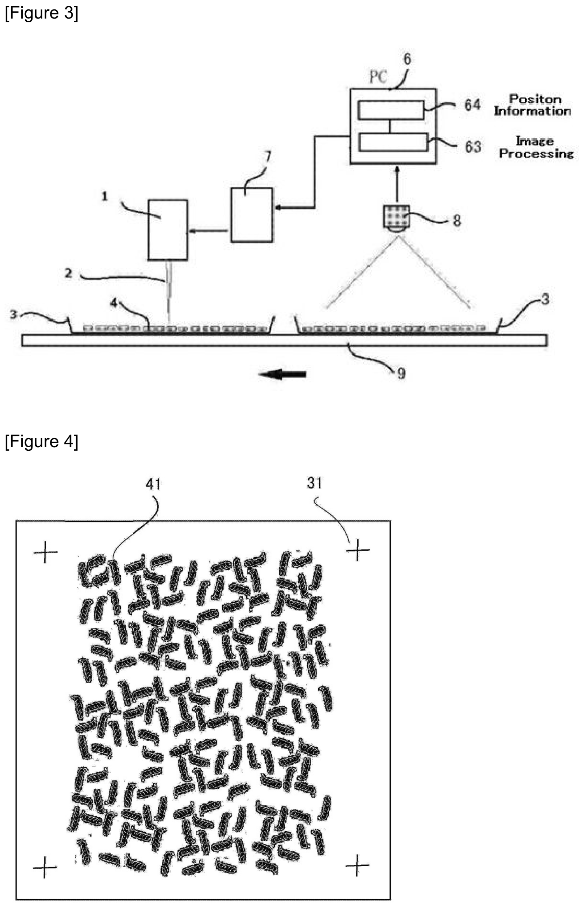

[FIG. 3] is a conceptual diagram when image processing is used in the method of the present invention, in which a CCD camera (8) is added to the laser marker in [FIG. 1]. Although the built-in camera of the laser marker can be used, it is preferable to install a small camera (CMOS camera) or a CCD camera (CCD sensor) independently of the commercially available laser marker or to attach it externally to the laser marker. The method of the present invention can be easily implemented by adding a CCD camera (8) to the laser marker shown in FIG. 1.

As shown in [Figure 3], an image acquired by the CCD camera (8) is sent to the PC (6), where it is processed, the position information of the insect (workpiece) is calculated, and the position information is sent to the controller (7) (Fig. 1) to control the movement of the laser beam from the head (1) of the laser marker. Some commercially available laser markers can import WORD data or bitmap data as is and perform laser marking. In such case, the movement of the laser beam can be controlled easily based on accurate position information simply by adding the image processing to the laser marker.

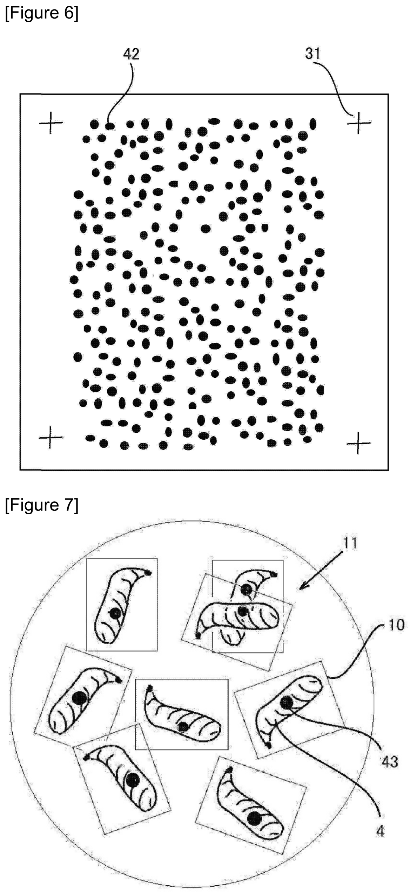

In practice, after taking an image of a group of housefly larvae (4) in a container, for example a tray (3), with a CCD camera (8), the tray (3) is moved under the head (1) of the laser marker. This movement may be automatic or manual. A support (9) for the tray (3) may be a conveyor belt and can be advanced in the direction of the arrow. The position markers (31) (see FIGS. 4 and 7) can be used as a reference position for the image taken by the CCD camera (8) and for the image taken by the position detection camera built in the head (1) of the laser marker.

The device of FIG. 3 can have further or additional means and devices. For example, it is possible to incorporate means to cool down and stop the movement of house fly larvae, such as freezing means, means to uniformly distribute the group of house fly larvae, such as a vibrating device, and an automatic loading and unloading device for the tray (3).

The image processing can be performed generally with simple software built into a PC (or added later). [FIG. 4] is a conceptual diagram of a screen obtained by simply performing image processing using binarization processing. In this case, the camera image of the house fly larva group obtained by the CCD camera (8) is sent to the PC (6), where image processing is performed to extract only the house fly larvae, as shown in Fig. 4 to obtain a binarized screen of a house fly larva (41). Such binarized positional information of the house fly larva group obtained in this way may be used as is with a laser marker. Preferably, the binarized position information is further processed to remove noise as necessary to calculate the position information of the insect (workpiece), and the position information is sent to the laser marker controller (7) in which the movement of the laser beam from the laser marker head (1) is controlled.

### Acquisition of more precise location information

If it is desired to irradiate a laser beam to a specific location on the insect (workpiece), for example, only the abdomen of a house fly, it is necessary to send more precise position information to the controller (7). In this case, Al image processing technology or the like can be used. Commercially available Al image processing software can be used for this processing.

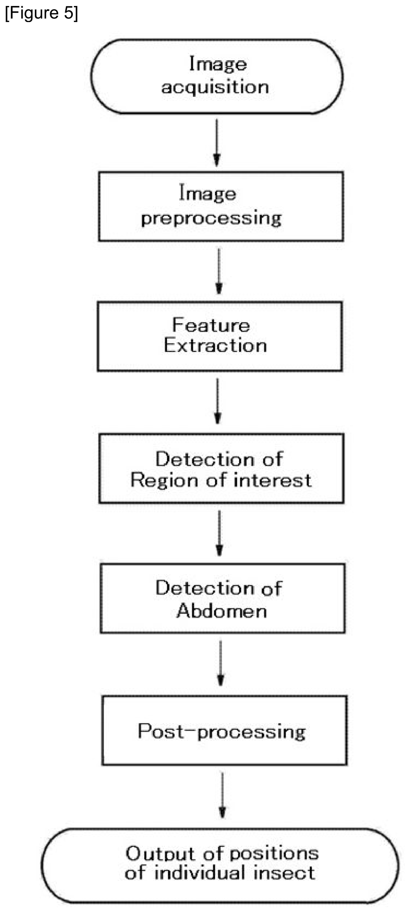

[FIG. 5] is a flowchart showing one example of basic operations for acquiring more precise positional information when the method of the present invention is implemented on house flies.
(1) "Image acquisition" uses a camera or the like to take an image of a group of houseflies.
(2) "Image preprocessing" performs processing such as house fly normalization and noise reduction.
(3) "Feature extraction" uses a pre-trained convolutional neural network (CNN), such as ResNet, MobileNet, or YOLO, to extract the features of the housefly.
(4) "Detection of region of interest" specifies a region in the image where the abdomen of the house fly may exist.
(5) "Abdominal Detection" uses additional neural networks or image processing techniques to further narrow down the detected region and specifically identify the abdomen of the house fly.
(6) "Post-processing" improves the results by filtering out false positives and adjusting bounding boxes.
(7) "Output" displays and saves the final result indicating the XY coordinate position of the housefly's abdomen in the image.

[FIG. 6] is a screen display of an example of the positions of abdomen of house flies (42) obtained from precise position information obtained according to the flowchart described in [FIG. 5]. The laser marker controller (7) controls the movement of the laser beam from the head (1) based on this position information.

[FIG. 7] is a conceptual diagram of image processing for detecting the "individual region" (10) of each house fly (4) and identifying its abdomen (43, black circle). In this case, it is also possible to decide in advance how to deal with house flies (11) that overlap each other.

### Induction of antimicrobial peptides

After laser irradiation, the house flies are left for 3 to 48 hours to induce antibacterial peptides in their bodies. To prevent pupation, house fly larvae are transferred for example to an environment at a wet bulb temperature of 20°C to 35°C and maintained for 3 to 48 hours to induce antibacterial peptides in the larvae themselves.

### Recovery of antibacterial peptides

After induction of antibacterial peptide, antibacterial peptide is collect from house flies. In practice, the tip of a house fly is cut, and the body fluid leaks from the cut and is collected in a tube on ice. The body fluid can be centrifuged at 100 g for 10 minutes to remove blood cells and the supernatant is collected and stored at -30°C.

### Measurement of antibacterial activity

According to the standard method, bacteria are plated on an agar medium, cultured, and antibacterial activity is measured to evaluate the ability to induce antibacterial peptides. The bacteria Staphylococcus aureus, Staphylococcus epidermidis, Escherichia coli, Pseudomonas aeruginosa, etc. can be selected.

Figures used in the above explanation are conceptual diagrams or partially enlarged diagrams, and their dimensions are not exact. Furthermore, the image processing and position information acquisition methods are not limited to those described above.

Examples of the present invention will be described below, but the present invention is not limited to the following examples.

### [Example]

### Example 1

The following equipment and methods were used.

### Laser equipment

Keyence Corporation ML-Z9620 CO₂ laser marker was used. This is a CO₂ laser marker with simultaneous scanning of 3 axes of X, Y, and Z. This machine can irradiate a working space of 100 x 100 mm or 300 x 300 mm with a laser beam of wavelength 10.6 µm and output of 30 W using scanning mode.

### Laser irradiation method

A glass tray (100 x 100 mm) was cooled to a temperature just before house fly larvae freeze (approximately 3°C). Then, about 1,000 house fly larvae were placed on it and were arranged on a flat surface so that the larva groups do not overlap. The movement of house fly larvae is significantly reduced on the cooled trays. A laser beam was irradiated onto the group of house fly larva by using the above CO₂ laser marker, with "scan printing" mode, with a marking pattern of 1 mm x 1 mm grid, the printing line segment length being set to 0.1 mm, and the scanning speed being set to 250 mm/s. The laser power was set to 80% of the output. An amount of heat added corresponds to 0.0096J.

### Induction of antimicrobial peptides

House fly larvae that have been irradiated with laser were left for 24 hours on a wet gauze containing physiological saline at a concentration close to the osmotic pressure of the larvae maintained at 20°C to 35°C to induce the peptide in the body of housefly larvae.

### Recovery of antibacterial peptides

After 24 hours, the tip of housefly larvae was cut with sharp scissors, and the body fluid leaked from the cut and was collected in a tube on ice. Approximately 500 µl of hemolymph was collected from approximately 500 larvae. After centrifuging the body fluid at 100 g for 10 minutes to remove blood cells, the supernatant was collected and stored at -30°C.

### Measurement of antibacterial activity

Staphylococcus aureus was cultured in LB medium and bacteria in the logarithmic growth phase (OD600 = 0.15-0.3) were used for measurement. Staphylococcus aureus in medium (2 µl) was mixed with 18 µl of body fluid sample. The bacteria/body fluid mixture was incubated at 30° C for 1 hour and then diluted with Mueller-Hinton II medium. The diluted measurement mixture (100 µl) was plated on an agar medium and cultured at 30° C for 1 day. After culturing, the colony forming units (CFU) of Staphylococcus aureus were determined.

The colony forming units (CFU) of Staphylococcus aureus in the diluted measurement mixture (Blank) was 167 x 10⁵ CFU/ml.

In contrast, when laser irradiation was performed using the method of the present invention, the colony forming unit (CFU) of Staphylococcus aureus was 0 (zero) CFU/ml, indicating that the growth of Staphylococcus aureus could be effectively suppressed.

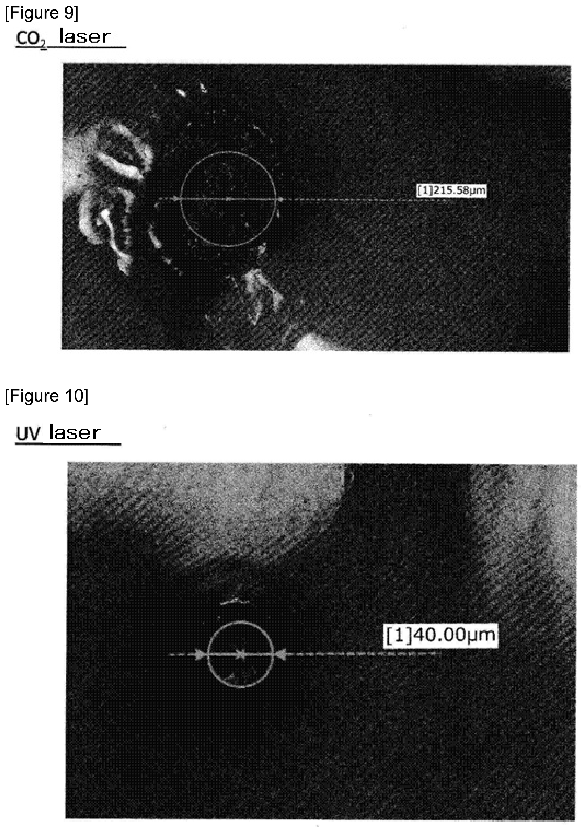

[FIG. 9] is a photograph showing that a hole of approximately 250 µm was formed in epidermis of a house fly larva when laser irradiation was performed in Example 1.

### Example 2

The same operations as in Example 1 were repeated, but the marking pattern was changed to a 1 mm x 4 mm grid.

In this case also, the colony forming unit (CFU) of Staphylococcus aureus was 0.5×10⁵ CFU/ml or less, indicating that the growth of Staphylococcus aureus could be effectively suppressed, similar to the results of Example 1.

### Example 3

The same operations as in Example 2 were repeated, but the laser beam irradiation mode was changed from the "scan printing" mode to the "fixed point printing" mode. The fixed point printing time was 1 ms, and an amount of heat added in this case was equivalent to 0.0240 J, which is about 2.5 times that of Example 2 in terms of the amount of heat.

In this case also, the colony forming unit (CFU) of Staphylococcus aureus was 2.3×10⁵ CFU/ml or less, indicating that, similar to the results of Example 2, the growth of Staphylococcus aureus could be effectively suppressed.

### Example 4

The same operations as in Example 3 were repeated, but the fixed-point printing interval was changed to a 1 mm x 1 mm grid.

In this case also, the colony forming unit (CFU) of Staphylococcus aureus was 0.5×10⁵ CFU/ml or less, indicating that, similar to the results of Example 3, the growth of Staphylococcus aureus could be effectively suppressed.

### Example 5

After laser irradiation, the larvae irradiated were left in a soil mixed with physiological saline for 24 hours. Thereafter, the same operations as in Example 4 were repeated.

It was shown that the effect of inhibiting the growth of Staphylococcus aureus decreased a little. In this case, the colony forming unit (CFU) of Staphylococcus aureus in this case was 22×10⁵ CFU/ml or less.

### Comparative examples 1 to 3

The same operation as in Example 3 was repeated using the laser irradiation device with a UV laser marker instead of the CO₂ laser marker.

The UV laser marker used was MD-U1020C manufactured by Keyence Corporation, and this UV laser marker can irradiate a laser beam with a wavelength of 335 nm and a printing output of 2.5 W. The printing mode in Comparative Examples 1 to 3 was the same "fixed point printing" mode as in Example 3.

The irradiation conditions and the obtained results (Staphylococcus aureus colony forming units (CFU)) are summarized in Table 1 below.

**[Table 1]**

| | **fixed point printing time ms** | **Heat J** | **irradiation interval** | **Colony forming units of Staphylococcus aureus x 10⁵ CFU / ml** |
|---|---|---|---|---|
| **Comparative 1** | **3** | **0.0060** | **1x1 mm** | **69** |
| **Comparative 2** | **3** | **0.0060** | **1x4 mm** | **61** |
| **Comparative 3** | **4** | **0.0080** | **1x1 mm** | **59** |

[Figure 10] is a photograph of an epidermis of house fly larva when the laser beam hits the house fly larva during UV laser irradiation in Comparative Example 3, showing that it is not possible to make holes in the epidermis of the house fly larva. In the case of UV laser, it can be irradiated with a narrow laser beam of about 40 µm, but the effect of inhibiting the growth of Staphylococcus aureus is low. The reason seems to be that they were unable to make holes in the epidermis of house fly larvae.

### Comparative examples 4 to 7

Comparative Example 4 is a "control" (described below).

In Comparative Example 5, an antibacterial peptide was induced in the body of a house fly larva by manually pricking the house fly larva with a needle using a conventional method without using a laser. A stainless steel needle with a diameter of 0.3 mm was used to pierce the abdomen of the house fly larva.

In Comparative Example 6, for comparison with Example 5, the larvae were left, after laser irradiation, in the soil mixed with physiological saline for 24 hours.

Comparative Example 7 is a "blank" (described below).

Induction of the antibacterial peptides was performed in the same manner as in Example 1, except that laser irradiation was not effected.

The irradiation conditions and the obtained results (colony forming units (CFU) of Staphylococcus aureus) of Comparative Examples 4 to 7 are summarized in Table 2 below.

**[Table 2]**

| | **Colony forming units of Staphylococcus aureus x 10⁵ CFU / ml** |
|---|---|
| **Comparative 4 (Control)** | **88** |
| **Comparative 5** | **57** |
| **Comparative 6** | **62** |
| **Comparative 7 (Blank)** | **167** |

"Control" (Comparative Example 4) in Table 2 refers to the case of house fly larvae that are neither irradiated with laser nor punctured with a needle to induce antibacterial peptides. The staphylococcus aureus colony forming units (CFU) was measured by the same method as in Example 1, and found that the CFU was 88 x 10⁵ CFU/ml.

"Blank" (Comparative Example 7) is the result when antibacterial activity was measured in the same manner as in Example 1, except that the body fluid of house fly larvae was not included. The colony forming units (CFU) of Staphylococcus aureus in this "blank" case was 167 x 10⁵ CFU/ml.

From the results in Table 2, the followings can be observed:
1) The effect of inhibiting the growth of Staphylococcus aureus according to the present invention (Examples 1 to 5) is significantly higher than that of the UV laser (Comparative Examples 1 to 3).
2) The effect of inhibiting the growth of Staphylococcus aureus according to the present invention (Examples 1 to 5) is higher than that of the conventional method (Comparative Examples 5 and 6). This indicates that "making a hole through the insect's epidermis with a laser beam" is necessary for the effect of inhibiting the growth of Staphylococcus aureus.
3) Even house fly larvae without any treatment (control of Comparative Example 4) have a certain effect of inhibiting the growth of Staphylococcus aureus compared to the blank (Comparative Example 7).

### [Explanation of symbols]

- 1: Head of a laser irradiation device
- 2: Laser beam
- 3: Tray
- 4: Housefly larva
- 6: PC
- 7: Controller
- 8: Camera
- 9: Support

## Claims

1. In a method for inducing antibacterial peptides in insects by damaging the insects, the method is **characterized in that** the damaging of the insects is performed by irradiation of a laser beam, in such a manner that the laser beam penetrates the insect's epidermis to make a hole in the insects without causing irreparable damage to the insect, so that antibacterial peptides is induced in the insects.

2. The method according to claim 1, wherein the insect is a house fly larva and the laser is a CO₂ laser.

3. The method according to claim 1, wherein the diameter of the hole penetrating the epidermis of the house fly larva is in a range of 1 to 300 µm.

4. The method according to claim 1, wherein the laser is irradiated to the insect larva under such a condition that movement of the insect larva is sealed by lowering a temperature just before freezing.

5. The method according to claim 2, wherein, after laser irradiation, the house fly larva irradiated with the laser is transferred to an environment of a bulb temperature of 20°C to 35°C for 3 hours to 48 hours to induce antibacterial peptides in the larva.

6. The method according to claim 4, wherein the house fly larvae is placed on a wet surface containing a liquid with a liquid having a near osmotic pressure maintained at 20°C to 35°C so that the house fly larvae is contacted with the liquid to inhibit pupation.

7. An apparatus for inducing antibacterial peptides in a body of insect by the method according to claim 1, comprising a CO₂ laser irradiation unit, a position information acquisition means for obtaining a position information of the insect, and a means for transmitting the insect position information obtained by the position information acquisition means to the insect position information.

8. The apparatus according to claim 7, comprising further means to prevent movement of the insect larvae by cooling the insect larvae to a temperature just below freezing.

9. Use of a CO₂ laser in a method to induce antibacterial peptides in the insect's body by making holes that penetrate the insect's epidermis.
